# EUROPÄISCHE PATENTSCHRIFT

(11) **EP 0 584 553 B1**
(45) Veröffentlichungstag und Bekanntmachung des Hinweises auf die Patenterteilung: **22.04.1998**
(21) Anmeldenummer: 93111948.1
(22) Anmeldetag: 26.07.1993
(51) Int. Cl.: A41B 13/04, A61F 5/44

(54) **Unterwäsche**
Undergarment
Sous-vêtement

(30) Priorität: 28.07.1992 JP 201453/92
(43) Veröffentlichungstag der Anmeldung: 02.03.1994
(73) Patentinhaber: UNI-CHARM CORPORATION, Kawanoe-shi Ehime-ken (JP)
(72) Erfinder: Fujioka, Yoshihisa, Mitoyo-gun, Kagawa-ken (JP); Mukai, Hirotomo, Kawanoe-shi, Ehime-ken (JP)
(74) Vertreter: Sperling, Rüdiger, Dipl.-Ing.

(56) Entgegenhaltungen:
- EP-A- 0 187 728
- EP-A- 0 452 951
- US-A- 3 225 918
- US-A- 4 959 059

## Beschreibung

Die vorliegende Erfindung betrifft Unterkleidung, umfassend einen Vorderteil und einen Hinterteil, wobei der Vorderteil und der Hinterteil entlang einander seitlich gegenüberliegenden Seiten offen sind und der Hinterteil an einander seitlich gegenüberliegenden Seiten mit Befestigungseinrichtungen versehen, ist, die zum Befestigen des Hinterteils am Vorderteil verwendet werden.

Ein typisches Beispiel für Wegwerfwindeln des offenen Typs mit Bandbefestigungseinrichtungen, die zum Befestigen eines Vorder- und Hinterteils aneinander in der Hüftlinie verwendet werden, ist im japanischen Patent Nr. JP-B-52-40267 [& US-A-3 860 003] aufgezeigt. Dieses Beispiel umfaßt eine flüssigkeitsdurchlässige Decklage, eine flüssigkeitsundurchlässige Außenlage und eine zwischen diese gelegte, flüssigkeitsabsorbierende Platte, wobei von Abschnitten der Deck- und der Außenlage, die sich über die seitlich einander gegenüberliegenden Seiten der Platte hinaus nach außen erstrecken, zwei seitliche Klappen gebildet werden und die jeweiligen seitlichen Klappen im Schrittbereich mit Ausschnitten versehen sind, die zur Bildung von Beinöffnungen bestimmt sind, um deren Umfang die jeweiligen seitlichen Klappen mit elastischen Elementen versehen sind, die zum Abdichten der Seitenklappen um das jeweilige Bein des Trägers dienen, und wobei der Hinterteil an einander seitlich gegenüberliegenden Seiten mit Befestigungsbändern versehen ist, die zum Befestigen des Hinterteils am Vorderteil verwendet werden.

Bei der in vorstehend genanntem japanischen Patent Nr. JP-B-52-40267 aufgezeigten Windel verringern in typischer Weise die in den einander gegenüberliegenden Seiten des Schrittbereichs zum verbesserten Sitz der Windel am Körper des Trägers ausgebildeten Ausschnitte notwendigerweise die Breite des Schrittbereichs und es ist praktisch unmöglich, daß der Schrittbereich die Oberschenkel des Trägers vollständig umgibt. Der auf diese Weise breitenreduzierte Schrittbereich verringert unvermeidbar die Fähigkeit des Schrittbereichs, flüssige Ausscheidungen aufzunehmen, und insbesondere treten flüssige Ausscheidungen direkt entlang einander gegenüberliegenden Seitenrändern des Schrittbereichs auf.

Allgemein verläuft bei der bekannten Windel des im vorstehend genannten japanischen Patent aufgezeigten Typs eine gewisse Faltlinie im Schrittbereich, entsprechend einer Grenzlinie des Vorder- und Hinterteils horizontal parallel zur Hüftlinie. Darüberhinaus weist die flüssigkeitsabsorbierende Platte sogenannte halbsteife Eigenschaften auf, da sie oftmals eine mehr oder weniger komprimierte Ansammlung von Faserpulpe sowie Seidenpapierlagen, die die Ober- und Unterfläche dieser Ansammlung bedecken, umfaßt. Dementsprechend kann der Schrittbereich der Windel sich nicht dem entsprechenden Bereich des Körper des Trägers anpassen, womit nicht nur dem Träger das Gefühl eines nicht passenden Kleidungsstückes vermittelt wird, sondern auch das Austreten von Ausscheidungen verursacht wird. Aus der EP-A-0 187 728 ist eine Windel des geschlossenen Typs bekannt, bei welcher das Vorderteil und das Hinterteil der Windel im Schrittbereich über eine Schweißlinie verbunden ist. Im Hinblick auf das vorstehend erwähnte Problem ist es Aufgabe der Erfindung, Unterkleidung mit einem Schrittbereich aufzuzeigen, der so ausgebildet ist, daß er Vorteile einer Windel des offenen Typs mit Vorteilen einer Windel des kurzen Höschentyps verbindet und dadurch die vorstehend genannten Nachteile der bekannten Windeln zu eliminieren.

Der Vorteil der Windel des offenen Typs liegt darin, daß die Windel einstellbar um die Hüftöffnung wie auch um die Beinöffnungen zusammengezogen werden kann und der Vorteil der Windel des kurzen Höschentyps liegt darin, daß sie Windelabschnitte aufweist, die wenigstens die Innenseite der Oberschenkel des Trägers vollständig bedecken, da die Beinabschnitte der kurzen Höschen und der Schrittbereich in größeren Abmessungen ausgeführt werden können als bei einer Windel des offenen Typs.

Die vorstehend genannte Aufgabe wird dadurch gelöst, daß der Vorderteil und der Hinterteil unmittelbar an ihren unteren Enden nur entlang einer konvex zur Hüftlinie des Vorderteils und des Hinterteils hin gekrümmten Schweißlinie miteinander zur Bildung eines Schrittbereich verschweißt sind.

Handelt es sich bei der Unterkleidung um eine Wegwerfwindel, so umfassen der Vorderteil und der Hinterteil jeweils eine flüssigkeitsdurchlässige Decklage, eine flüssigkeitsundurchlässige Außenlage und eine zwischen diese Decklage und Außenlage gelegte flüssigkeitsabsorbierende Platte, und der Hinterteil ist an einander seitlich gegenüberliegenden Seiten in der Ebene des Hüftbereichs mit Befestigungseinrichtungen versehen, wie zum Beispiel mit druckempfindlichem Klebeband. Handelt es sich bei der Unterkleidung um ein Windelhöschen, so umfassen der Vorderteil und der Hinterteil jeweils wenigstens eine flüssigkeitsundurchlässige Lage und sind an einander gegenüberliegenden Seiten in der Ebene der Hüftlinie in der Weise mit Befestigungseinrichtungen, wie zum Beispiel Velcro (Warenzeichen) versehen, daß diese Befestigungseinrichtungen so positioniert werden können, daß der Vorder- und Hinterteil aneinander befestigt werden können, wobei die einander gegenüberliegenden Seiten des ersteren die entsprechenden Seiten des letzteren überlappen. Vorzugsweise sind der Vorderteil und der Hinterteil entlang der Hüftlinie und um ein Paar von Beinöffnungen mit in Umfangsrichtung dehnbaren elastischen Elementen versehen.

Bei der gemäß der Erfindung wie vorstehend beschrieben aufgebauten Unterkleidung wird die von den unteren Enden des Vorderteils und des Hinterteils, die durch die konvex gekrümmte Schweißlinie miteinander verschweißt sind, gebildete Innenfläche des Schrittbereichs zuverlässig im Schritt des Trägers mittig ausgerichtet und die einander seitlich gegenüberliegenden Seiten des Schrittbereichs bedecken jeweils zuverlässig wenigstens die Innenseiten der Oberschenkel des Trägers. Auf diese Weise bedeckt der Schrittbereich der Unterkleidung im wesentlichen den gesamten Schrittbereich des Trägers.

Die Erfindung wird als Beispiel unter Bezug auf die beiliegenden Figuren beschrieben, wobei:
- Fig. 1: eine Vorderansicht einer Ausführungsform der gemäß der Lehre der Erfindung aufgebauten Windel zeigt;
- Fig. 2: eine schematische Schnittdarstellung entlang einer Linie A-A in Fig. 1 zeigt;
- Fig. 3: eine schematische Schnittdarstellung entlang einer Linie B-B in Fig. 1 zeigt; und
- Fig. 4 (A), (B) und (C): Vorderansichten sind, die beispielhaft die konvex gekrümmte Schweißlinie darstellen.

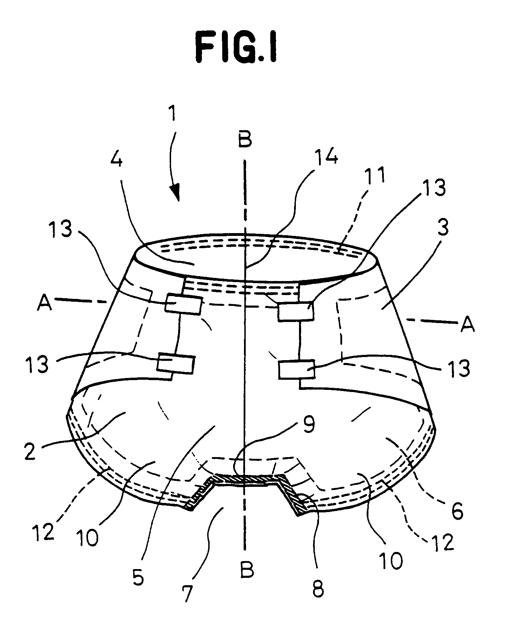

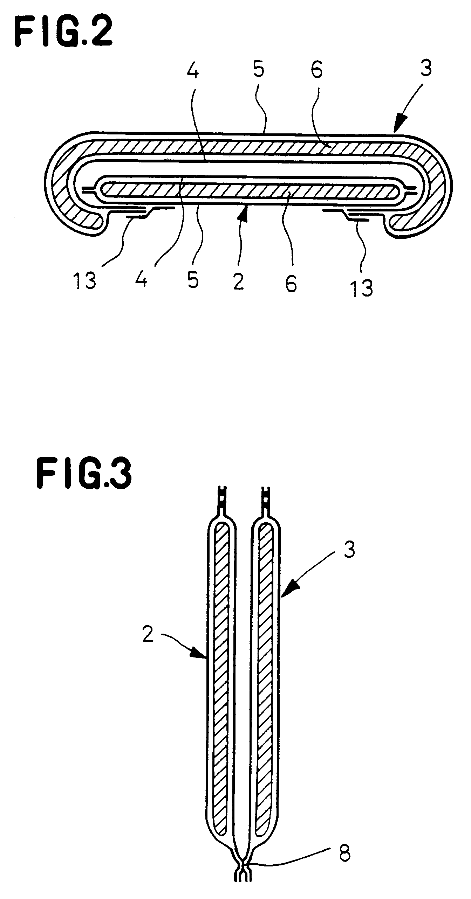

Wie in Fig. 1 bis 3 dargestellt, umfaßt eine Windel 1 allgemein einen Vorderteil 2 und einen Hinterteil 3. Der Hinterteil 3 ist in Querrichtung größer bemessen als der Vorderteil 2. Der Vorderteil 2 und der Hinterteil 3 umfassen jeweils eine flüssigkeitsdurchlässige Decklage 4, eine flüssigkeitsundurchlässige Außenlage 5 und eine zwischen die Decklage 4 und die Außenlage 5 gelegte flüssigkeitsabsorbierende Platte 6.

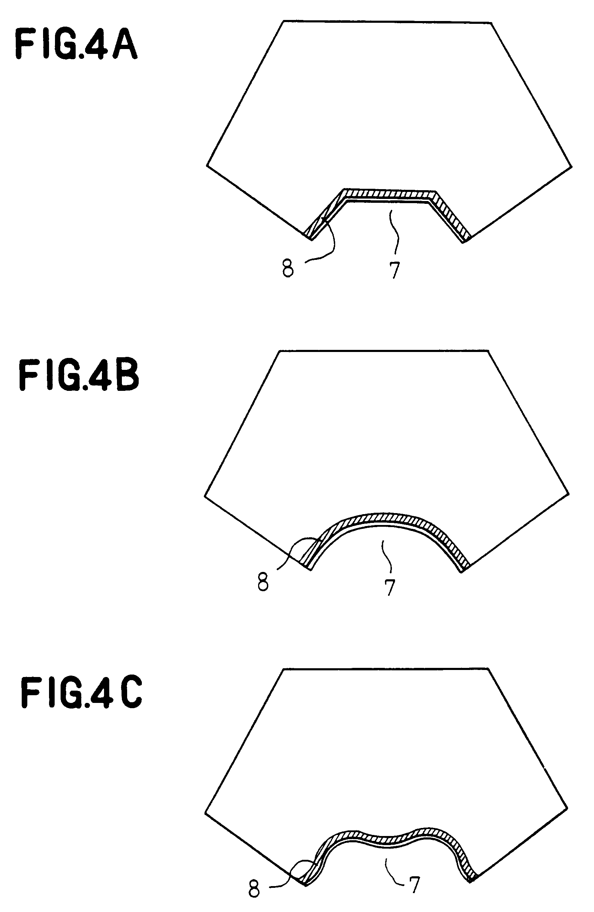

Die unteren Enden des Vorder- und Hinterteils 2, 3 sind im Mittelabschnitt mit konvex auf die Hüftlinien des Vorderteils 2 und des Hinterteils 3 zu gekrümmten Ausschnitten 7 versehen. Der Vorder- und Hinterteil 2, 3 sind entlang einer thermischen oder Ultraschall-Schweißlinie 8, die parallel zu den Ausschnitten 7 verläuft, miteinander verschweißt, so daß schmale Ränder der Ausschnitte 7 unverschweißt belassen werden. Die Schweißlinie 8 kann eine Kurve beschreiben, beispielsweise wie durch Fig. 4 (A), (B) oder (C) dargestellt. Größen, Formen und Kurvenradien der Schweißlinie 8 können in geeigneter Weise in Abhängigkeit davon gewählt werden, ob die Windel für einen Erwachsenen oder für ein Baby bestimmt ist, sofern die Schweißlinie 8 konvex auf die Hüftlinien des Vorder- und Hinterteils 2, 3 zu gekrümmt ist und wenigstens die Innenseiten von jeweiligen Beinöffnungen 10 sich nach unten über einen Scheitelpunkt 9 der konvex gekrümmten Schweißlinie 8 hinaus erstrecken.

In Umfangsrichtung dehnbare elastische Elemente 11, 12 sind zwischen die jeweiligen Ränder der Deck- und Außenlage 4, 5 gelegt, die um die Hüftöffnung und die Beinöffnungen jeweils über die Platte 6 hinaus verlaufen, und diese Ränder sind durch Heißschmelzkleber oder durch Schweißmittel miteinander verbunden.

Einander seitlich gegenüberliegende Seitenränder der Decklage 4 und der Außenlage 5, die über die Platte hinaus reichen, sind ebenfalls in derselben Weise miteinander verbunden und der Hinterteil 3 ist an einander seitlich gegenüberliegenden Seitenrändern mit mehreren Befestigungseinrichtungen 13 versehen, die jeweils ein auf einer Seite mit einem druckempfindlichen Klebstoff befestigtes Befestigungsband umfassen, durch das die seitlich einander gegenüberliegenden Seiten des Hinterteils 3 an den entsprechenden Seiten des Vorderteils 2 befestigt werden.

Während die einander seitlich gegenüberliegenden Seitenränder und unteren Ränder um die jeweiligen Beinöffnungen des Vorder- und Hinterteils 2, 3 so dargestellt sind, daß sie weder parallel noch senkrecht zu einer vertikalen Achse 14 verlaufen, können im Rahmen der Erfindung diese Seitenränder parallel mit der vertikalen Achse 14 verlaufen und die unteren Ränder um die jeweiligen Beinöffnungen können senkrecht zur vertikalen Achse 14 verlaufen.

Die dergestalt aufgebaute Windel 1 ist an den einander seitlich gegenüberliegenden Seiten daher an der Hüftöffnung wie auch an den Beinöffnungen geöffnet und nach dem Aufrichten unter Verwendung der Befestigungseinrichtungen, wie in Fig. 1 dargestellt, sind die einander seitlich gegenüberliegenden Seiten geschlossen und in der Folge werden die Hüftöffnung wie auch die Beinöffnungen ebenfalls geschlossen. Die Größen dieser Öffnungen sind von der Überlappungsbreite des Vorderteils 2 mit dem Hinterteil 3 abhängig und die Überlappungsbreite ist wiederum von der Hüft- bzw. Bein- (Oberschenkel)größe des einzelnen Trägers abhängig.

Die Einzelteile der Windel 1 können aus allgemein in bekannten Windeln verwendeten Materialien hergestellt sein. Beispielsweise können die Decklage 4 aus Vliesstoff, die Außenlage 5 aus Kunststoffolie, die Platte 6 aus Faserpulpe, gemischt mit hochabsorbierendem Polymer, die elastischen Elemente 11, 12 aus natürlichem oder synthetischem Gummi und der Träger der Befestigungseinrichtungen 13 aus qualitativ hochwertigem Papier oder Schichtstoff aus Vliesstoff und Kunststoffolie hergestellt sein. Es sei angemerkt, daß die Unterkleidung aus wenigstens einer flüssigkeitsundurchlässigen Lage, wie zum Beispiel wasserdicht ausgerüstetem Gewebe hergestellt sein kann, wenn es sich bei der Unterkleidung um ein Windelhöschen handelt.

Bei der gemäß der Lehre der Erfindung wie vorstehend beschrieben aufgebauten Unterkleidung führt die Bildung der Ausschnitte an den einander gegenüberliegenden Seiten des Schrittbereichs zur Bildung der Beinöffnung niemals zu einer unannehmbar schmalen Breite des Schrittbereichs, da die zur Hüftlinie konvex gekrümmte Schweißlinie die Dimensionierung der Breite des Schrittbereichs zwischen den einander gegenüberliegenden Seitenrändern des Schrittbereichs in angemessener Größe erlaubt. Darüber hinaus erlaubt es die Bildung einer derartigen Schweißlinie, daß der Schrittbereich wenigsten die Innenseiten der jeweiligen Oberschenkel bedeckt, womit die Nachteile von bekannter Unterkleidung, wie zum Beispiel Wegwerfwindeln, vermieden sind.

## Patentansprüche

1. Unterkleidung, umfassend einen Vorderteil (2) und einen Hinterteil, wobei der Vorderteil (2) und der Hinterteil (3) entlang einander seitlich gegenüberliegenden Seiten offen sind und der Hinterteil (3) an einander seitlich gegenüberliegenden Seiten mit Befestigungseinrichtungen (13) versehen ist, die zum Befestigen des Hinterteils (3) am Vorderteil (2) verwendet werden, **dadurch gekennzeichnet**, daß der Vorderteil (2) und der Hinterteil (3) unmittelbar an ihren unteren Enden nur entlang einer konvex zur Hüftlinie des Vorderteils (2) und des Hinterteils (3) hin gekrümmten Schweißlinie (8) miteinander zur Bildung eines Schrittbereichs verschweißt sind.

2. Unterkleidung nach Anspruch 1, wobei der Vorderteil (2) und der Hinterteil (3) jeweils eine flüssigkeitsdurchlässige Decklage (4), eine flüssigkeitsundurchlässige Außenlage (5) und eine zwischen die Deck- und Außenlage gelegte flüssigkeitsabsorbierende Platte (6) umfassen.

3. Unterkleidung nach Anspruch 1, wobei der Vorderteil (2) und der Hinterteil (3) jeweils wenigstens eine flüssigkeitsundurchlässige Lage umfassen.

4. Unterkleidung nach Anspruch 1, wobei der Hinterteil (3) in Querrichtung größere Abmessungen hat als der Vorderteil (2).

5. Unterkleidung nach Anspruch 1 oder 2, wobei der Vorderteil (2) und der Hinterteil (3) außerhalb des durch die konvex gekrümmte Schweißlinie (8) gebildeten Schrittbereichs teilweise ausgeschnitten sind.

6. Unterkleidung nach Anspruch 1 oder 2, wobei der Vorderteil (2) und der Hinterteil (3) entlang der Hüftlinie und um ein Paar von Beinöffnungen (10) mit in Umfangsrichtung dehnbaren elastischen Elementen (11,12) versehen sind.

## Claims

1. Undergarment, comprising a front part (2) and a rear part, the front part (2) and the rear part (3) being open along sides which are laterally opposite each other and the rear part (3) being provided on sides which are laterally opposite each other with fastening means (13) which are used for fastening the rear part (3) to the front part (2), characterized in that the front part (2) and the rear part (3) are welded to each other directly at their lower ends only along a welding line (8) curved convexly with respect to the hip line of the front part (2) and of the rear part (3) to form a crotch region.

2. Undergarment according to Claim 1, the front part (2) and the rear part (3) in each case comprising a liquid-permeable liner (4), a liquid-impermeable outer layer (5) and a liquid-absorbent sheet (6) placed between the liner and the outer layer.

3. Undergarment according to Claim 1, the front part (2) and the rear part (3) in each case comprising at least one liquid-impermeable layer.

4. Undergarment according to Claim 1, the rear part (3) having greater dimensions in the transverse direction than the front part (2).

5. Undergarment according to Claim 1 or 2, the front part (2) and the rear part (3) being partially cut out outside the crotch region formed by the convexly curved welding line (8).

6. Undergarment according to Claim 1 or 2, the front part (2) and the rear part (3) being provided along the hip line and around a pair of leg openings (10) with elastic elements (11, 12) which can stretch in the circumferential direction.

## Revendications

1. Sous-vêtement, comprenant une partie avant (2) et une partie arrière, la partie avant (2) et la partie arrière (3) étant ouvertes le long de côtés latéraux opposés, et la partie arrière (3) étant pourvue, sur des côtés latéraux opposés mutuellement, d'éléments de fixation (13) utilisés pour fixer la partie arrière (3) à la partie avant (2), caractérisé en ce que la partie avant (2) et la partie arrière (3) sont soudées immédiatement à leurs extrémités inférieures et seulement le long d'une ligne de soudure (8) s'incurvant avec une courbure convexe vers la ligne de hanches de la partie avant (2) et de la partie arrière (3), pour former une zone d'entrejambe.

2. Sous-vêtement selon la revendication 1, dans lequel la partie avant (2) et la partie arrière (3) comprennent, chacune, une mince couche de revêtement (4) perméable aux liquides, une mince couche extérieure (5) imperméable aux liquides, et une couche plus épaisse (6) absorbant les liquides, interposée entre les couches de revêtement et extérieure.

3. Sous-vêtement selon la revendication 1, dans laquelle la partie avant (2) et la partie arrière (3) comportent, chacune, au moins une couche imperméable aux liquides.

4. Sous-vêtement selon la revendication 1, dans lequel la partie arrière (3) est de plus grandes dimensions, dans le sens transversal, que la partie avant (2).

5. Sous-vêtement selon l'une des revendications 1 ou 2, dans lequel la partie avant (2) et la partie arrière (3) sont partiellement découpées à l'extérieur de la zone d'entrejambe formée par la ligne de soudure (8) d'incurvation convexe.

6. Sous-vêtement selon l'une des revendications 1 ou 2, dans lequel la partie avant (2) et la partie arrière (3) sont munies d'éléments élastiques (11, 12) extensibles dans le sens périphérique, le long de la ligne de hanches et sur le pourtour de deux ouvertures de passage des jambes (10).
